# EUROPÄISCHE PATENTSCHRIFT

(11) **EP 0 981 969 B1**
(45) Veröffentlichungstag und Bekanntmachung des Hinweises auf die Patenterteilung: **03.01.2007**
(21) Anmeldenummer: 99116474.0
(22) Anmeldetag: 21.08.1999
(51) Int. Cl.: A23L 1/302, A23K 1/16, A23L 1/275, A23L 1/29, A61K 31/07

(54) **Carotinoid-Formulierungen, enthaltend ein Gemisch aus Beta-Carotin, Lycopin und Lutein**
Carotinoid compositions comprising a mixture of beta-carotene, lycopene and lutein
Composition de caroténoides compenant un mélange de beta-carotène, lycopène et lutéine

(30) Priorität: 26.08.1998 DE 19838636
(43) Veröffentlichungstag der Anmeldung: 01.03.2000
(73) Patentinhaber: BASF Aktiengesellschaft, 67056 Ludwigshafen (DE)
(72) Erfinder: Runge, Frank, Dr., 67133 Maxdorf (DE); Holm-Hansen, Jan, 3450 Alleroed (DK); Michelsen, Birgit, FRB (DK)

(56) Entgegenhaltungen:
- WO-A-96/19215
- WO-A-96/19217
- WO-A-97/15554
- SOLGAR "LUTEIN LYCOPENE CAROTENE COMPLEX VEGICAPS", XP002125651 Retrieved from the Internet: <URL:http://www.solgar.com/online_referenc e/beta_carotene/lutein.html>
- NATURMED ESSENTIALS "EYE FACTORS", [Online] XP002125652 Retrieved from the Internet: <URL:http://naturemed.com/ageless/ageless_ P/eyefactors.htm>
- ONCCOR "SIMONE PROTECTIVE HEALTHCARE", [Online] XP002125653 Retrieved from the Internet: <URL:http://www.drsimone.com/spcc/store/on ccor.htm>
- "BETATENE - MIXED CAROTINOIDS THE BODY NEEDS!", [Online] XP002125654 Retrieved from the Internet: <URL:http://www.idealink.com/lisa/betatene .htm>
- WEBVITAMINS "TWINLAB MULTI VITAMINS AND MINERALS", [Online] XP002125655 Retrieved from the Internet: <URL:http://www.webvitamins.com/TwinLabs/m ulti.htm>
- SOLGAR ONLINE REFERENCE "ADVANCED CAROTINOID COMPLEX SOFTGELS", [Online] XP002125656 Retrieved from the Internet: <URL:http://www.solgar.com/online_referenc e/beta_carotene/adv_car_comp_soft.html>
- VERIS "TYPICAL CAROTINOID CONTENT OF SELECTED FRUITS AND VEGETABLES", [Online] XP002125657 Retrieved from the Internet: <URL:http://veris-online.org/catyp2fb.htm>

## Beschreibung

Die Erfindung betrifft Carotinoid-Formulierungen, enthaltend ein Gemisch aus β-Carotin, Lycopin und Lutein, dadurch gekennzeichnet, dass der phosphorgehalt der Formulierung kleiner 2,0 Gew.-%, gew.-%, bezogen auf die Gesamtmenge des Gemisches aus β-Carotin, Lycopin und ist.

Carotinoide bilden eine Gruppe von Farbpigmenten mit gelber bis roter Farbtonnuance, die in der Natur weit verbreitet vorkommen und vielen Nahrungsmitteln ihre charakteristische Färbung verleihen.

Epidemiologische Studien haben außerdem gezeigt, daß ein häufiger und regelmäßiger Verzehr von Carotinoid-haltigem Obst und Gemüse das Risiko chronischer Erkrankungen, u.a. Herz- und Kreislauferkrankungen, mindert sowie einen positiven Einfluß auf die Krebsprävention ausübt.

Diese Schutzfunktion der Carotinoide wird sowohl in ihrer Wirkung als Antioxidants, als auch, wie im Falle von β-Carotin, in ihrer Pro-Vitamin-A-Aktivität gesehen.

Besonderes Interesse gilt in diesem Zusammenhang den drei Carotinoiden β-Carotin, Lycopin und Lutein, die weit verbreitet in Lebensmitteln wie z.B. Tomaten, Karotten, Spinat, Paprika und Broccoli vorkommen [siehe Journal of the American Dietetic Association, 97(9), 991-996 (1997)]. Insbesondere die Mischung dieser drei Carotinoide stellt ein System mit besonderen antioxidativen Eigenschaften dar.

Von Ernährungswissenschaftlern wird aus diesem Grund die Empfehlung für eine zusätzliche, präventive Zuführung antioxidativ wirkender Vitamine und Carotinoide gegeben. Der Pharma- und Lebensmittelmarkt bietet daher für den Verbraucher eine Vielzahl solcher "Zellschutzpräparate" an.

Formulierungen für Lebensmittelzubereitungen oder Nahrungsergänzungsmittel, die speziell eine Kombination aus β-Carotin, Lycopin und Lutein in hohen Konzentrationen und hoher Reinheit enthalten, sind jedoch bis dato nicht bekannt.

So ist Lycopin beispielsweise unter dem Namen Lyc-O-Mato^{®} (Fa. LycoRed, Israel) als 6 %ige ölige Dispersion erhältlich. Es wird gemäß WO 97/48287 als natürliches Carotinoid aus Tomaten extrahiert. Aufgrund des hohen Phospholipidanteils im Lyc-O-Mato^{®}, verbunden mit einer hohen Viskosität der öligen Dispersion, sind die anwendungstechnischen Eigenschaften dieser Formulierung nicht immer zufriedenstellend. Insbesondere für die Herstellung von hochkonzentrierten Carotinoid-haltigen Gelatinekapseln ist die Verwendung von Lyc-O-Mato^{®} unbefriedigend.

US 5,382,714 und US 5,648,564 beschreiben Verfahren zur Isolierung von Lutein aus dem Ölextrakt von Ringelblumen sowie die Verwendung des so gewonnenen Luteins als Lebensmittelfarbstoff und als Antioxidants in der Krebsprophylaxe. Zu Kombinationen aus β-Carotin, Lycopin und Lutein werden in diesen Patentschriften keine Hinweise geliefert.

Es war nun die Aufgabe, stabile Formulierungen einer Dreier-Kombination von Carotinoiden, bestehend aus β-Carotin, Lycopin und Lutein bereitzustellen, die die oben genannten Nachteile des Standes der Technik nicht aufweisen.

Diese Aufgabe wurde erfindungsgemäß gelöst durch Carotinoid-Formulierungen, enthaltend ein Gemisch aus β-Carotin, Lycopin und Lutein, dadurch gekennzeichnet, dass der Phosphorgehalt der Formulierung kleiner 2,0 Gew.-% bezogen auf die Gesamtmenge des Gemisches aus β-carotin, Lycopin und Lutein ist.

Mit dem Begriff "Carotinoid-Formulierungen" sind im Rahmen dieser Erfindung sowohl Lösungen, Solubilisate und Dispersionen, wie Emulsionen oder. Suspensionen als auch daraus hergestellte Trokkenpulver von Carotinoiden gemeint. Bevorzugte Formulierungen sind Dispersionen wie Emulsionen und Suspensionen, insbesondere ölhaltige Suspensionen.

Das Mengenverhältnis der in dem Gemisch vorliegenden Carotinoide liegt bei 1 Teil β-Carotin, 0,05 bis 20 Teile Lycopin und 0,05 bis 20 Teile Lutein, bevorzugt bei 1 Teil β-Carotin, 0,1 bis 5 Teile Lycopin und 0,1 bis 5 Teile Lutein, besonders bevorzugt bei 1 Teil β-Carotin, 0,2 bis 2 Teile Lycopin und 0,1 bis 2 Teile Lutein, ganz besonders bevorzugt bei 1 Teil β-Carotin, 0,3 bis 1,2 Teile Lycopin und 0,1 bis 0,5 Teile Lutein.

Der Gehalt an β-Carotin, Lycopin und Lutein, in Form der erfindungsgemäßen Kombination, liegt in den Formulierungen im allgemeinen zwischen 0,1 und 40 Gew.-%, bevorzugt zwischen 5 und 35 Gew.-%, besonders bevorzugt zwischen 10 und 33 Gew.-%, ganz besonders bevorzugt zwischen 15 und 32 Gew.-%, bezogen auf die Gesamtmenge der Formulierung.

Die Carotinoid-Formulierungen zeichnen sich weiterhin dadurch aus, daß der Phosphorgehalt in den Formulierungen kleiner 2,0 Gew.-%, vorteilhaft kleiner 1,0 Gew.-%, bevorzugt kleiner 0,5 Gew.-%, besonders bevorzugt kleiner 0,1 Gew.-%, ganz besonders bevorzugt kleiner 0,02 Gew.-%, bezogen auf die Gesamtmenge des Gemisches aus β-Carotin, Lycopin und Lutein ist.

Mit dem geringen Phosphorgehalt ist gleichzeitig auch ein geringer Anteil an Phospholipiden verbunden, wodurch die anwendungstechnischen Eigenschaften der Formulierungen, wie beispielsweise die Fließfähigkeit ölhaltiger Dispersionen besonders bei niedrigen Temperaturen verbessert werden. Die erfindungsgemäßen Dispersionen sind bereits bei Temperaturen zwischen 5°C und 40°C so niedrigviskos, daß in der weiteren Verarbeitung, z.B. bei der Abfüllung in Gelatinekapseln, auf das Erhitzen der Carotinoiddispersion (zur Viskositätserniedrigung) verzichtet werden kann. Damit können unerwünschte Aktivitätsverluste durch chemischen bzw. thermischen Abbau der Carotinoide vermieden werden.

Die für die Herstellung der Formulierungen verwendeten Carotinoide werden bevorzugt in Form ihrer Kristalle mit einer Reinheit von größer 75%, bevorzugt größer 90%, besonders bevorzugt größer 95%, ganz besonders bevorzugt größer 98% eingesetzt. Dabei ist es möglich, β-Carotin, Lycopin und Lutein sowohl aus natürlichen Quellen, als auch bevorzugt synthetisch hergestellte Carotinoide, insbesondere synthetisch hergestelltes β-Carotin und Lycopin einzusetzen. So ist beispielsweise das verwendete β-Carotin bzw. Lycopin erhältlich nach einem der Verfahren gemäß EP-A-382067 oder EP-A-000140.

Neben dem o.g. Gemisch aus β-Carotin, Lycopin und Lutein können die erfindungsgemäßen Carotinoid-Formulierungen mindestens einen weiteren Wirkstoff in Konzentrationen von 0,01 bis 40 Gew.-%, bevorzugt 0,1 bis 30 Gew.-%, besonders bevorzugt in Konzentrationen von 0,5 bis 20 Gew.-% enthalten.

### Dabei kann es sich beispielsweise um folgende Wirkstoffe handeln:

Weitere Carotinoide, wie z.B. Bixin, Zeaxanthin, Cryptoxanthin, Citranaxanthin, Canthaxanthin, β-Apo-4-carotinal, β-Apo-8-carotinal, β-Apo-8-carotinsäureester, Astaxanthin, einzeln oder als Mischung.

Vitamine, z.B. Vitamin A, Vitamin A-Acetat, Vitamin A-Palmitat, Riboflavin, Vitamin B₁₂, Ascorbinsäure, Ascorbylpalmitat, Nicotinsäure, Nicotinsäureamid, Pyridoxin Hydrochlorid, Vitamin D₃, Tocopherol, Tocopherolacetat, Tocopherolpalmitat, Tocotrienol, Vitamin K, Thiamin, Calcium-Pantothenat, Biotin, Liponsäure, Folsäure, Folsäurederivate wie Tetrahydrofolsäure, 5-Methyl-tetrahydrofolsäure, 10-Formyl-tetrahydrofolsäure oder 5-Formyl-tetrahydrofolsäure.

Verbindungen mit Vitamin- oder Coenzymcharakter, z.B. Cholinchlorid, Carnitin, Taurin, Kreatin, Ubichinone, S-Methylmethionin, S-Adenosylmethionin.

Mehrfach ungesättigte Fettsäuren, z.B. Linolsäure, Linolensäure, Arachidonsäure, Eicosapentaensäure, Docosahexaensäure.

Bestandteile des Knoblauchs, z.B. Diallylthiosulfinat, S-Allylcysteinsulfoxid, Vinyldithiine, Ajoen.

Allithiamine wie Benfotiamin, Fursultiamin, Octotiamin oder Bentiamin.

Glutathion und dessen Ester, wie z.B. GSH-monomethylester, GSHdimethylester, GSH-monoethylester, GSH-diethylester.

Je nach Art der Formulierung können diese neben den Carotinoiden mindestens einen weiteren Hilfs- oder Zusatzstoff, wie z.B. Öle, Schutzkolloide, Weichmacher, Antioxidantien und/oder Emulgatoren enthalten.

Im Falle einer Dispersion, insbesondere im Falle einer Suspension oder Emulsion, ist es vorteilhaft, zusätzlich ein physiologisch zugelassenes Öl wie beispielsweise Sesamöl, Maiskeimöl, Baumwollsaatöl, Sojabohnenöl oder Erdnußöl, Ester mittelkettiger pflanzlicher Fettsäuren sowie außerdem Fischöle wie beispielsweise Makrelen-, Sprotten- oder Lachsöl zu verwenden.

Als Schutzkolloide werden beispielsweise Gelatine, Fischgelatine, Stärke, Dextrin, Pflanzenproteine, Pektin, Gummi-Arabikum, Kasein, Kaseinat oder Mischungen davon verwendet. Es können aber auch Polyvinylalkohol, Polyvinylpyrrolidon, Methylcellulose, Carboxymethylcellulose, Hydroxypropylcellulose und Alginate eingesetzt werden. Bezüglich näherer Einzelheiten wird auf R.A. Morton, Fat Soluble Vitamins, Intern. Encyclopedia of Food and Nutrition, Bd.9, Pergamon Press 1970, S. 128-131, verwiesen. Zur Erhöhung der mechanischen Stabilität beispielsweise des Trockenpulvers ist es zweckmäßig, dem Kolloid einen Weichmacher zuzusetzen, wie Zucker oder Zuckeralkohole, z.B. Saccharose, Glucose, Lactose, Invertzucker, Sorbit, Mannit oder Glycerin.

Das Verhältnis Schutzkolloid und Weichmacher zu Carotinoid wird im allgemeinen so gewählt, daß als Endprodukt eine Formulierung erhalten wird, die neben den oben genannten Carotinoiden 10 bis 50 Gew.-% eines Schutzkolloids, 20 bis 70 Gew.-% eines Weichmachers, alle Prozentangaben bezogen auf die Trockenmasse der Formulierung, sowie gegebenenfalls untergeordnete Mengen eines Stabilisators enthält.

Zur Erhöhung der Stabilität des Wirkstoffes gegen oxidativen Abbau ist es vorteilhaft, Stabilisatoren wie α-Tocopherol, t-Butylhydroxy-toluol, t-Butylhydroxyanisol, Ascorbinsäure oder Ethoxyquine zuzusetzen.

Als Emulgatoren bzw. Solubilisatoren können im Falle von Emulsionen und daraus hergestellten Trockenpulvern sowie im Falle von Solubilisaten beispielsweise Ascorbylpalmitat, Polyglycerin-Fettsäureester, Sorbitan-Fettsäureester, Propylenglycol-Fettsäureester oder Lecithin in einer Konzentration von 0 bis 200 Gew.%, vorzugsweise 10 bis 150 Gew.%, besonders bevorzugt 20 bis 80 Gew.%, bezogen auf die Carotinoide, verwendet werden.

Die Herstellung der Carotinoid-Formulierungen erfolgt in an sich bekannter Weise. So lassen sich beispielsweise Solubilisate bzw. Emulsionen gemäß EP-A-0 055 817 oder EP-0 479 066 bzw. EP-A-0 551 638 herstellen. Die Herstellung von Carotinoid-Dispersionen und deren Überführung in ein Trockenpulver ist u.a. beschrieben in EP-A-0 498 824 und EP-A-0 410 236.

Die bevorzugten ölhaltigen Carotinoid-Dispersionen lassen sich in an sich bekannter Weise beispielsweise durch Mahlen der kristallinen Carotinoide in einem physiologisch zugelassenen Öl mittels einer Kugelmühle herstellen.

Die erfindungsgemäßen Carotinoid-Formulierungen eignen sich u.a. als Zusatzstoff zur Färbung von Lebensmittelzubereitungen, insbesondere von Getränkezubereitungen, als Mittel für die Herstellung pharmazeutischer und kosmetischer Zubereitungen sowie für die Herstellung von Nahrungsergänzungspräparaten im Human- und Tierbereich.

Für die Verwendung der erfindungsgemäßen Carotinoid-Formulierungen zum Färben von Lebensmitteln eignen sich prinzipiell alle der oben genannten Formulierungsarten. So können zum Färben von Getränken beispielsweise Emulsionen, Solubilisate oder auch wasserdispergerbare Trockenpulver verwendet werden, in denen Mischungen von β-Carotin, Lycopin und Lutein in den bereits oben genannten Konzentrationen vorliegen.

Auch die ölhaltigen Dispersionen haben schon in geringen Konzentrationen eine starke Farbkraft. Sie sind geeignet zur Färbung von Ölen und Fetten sowie von Lebensmitteln wie Margarine, Butter, Käsezubereitungen, Speiseeis, Suppen, Soßen und Eiprodukten durch Zugabe zur öligen Phase.

Es ist aber auch möglich Trockenpulver, die die erfindungsgemäßen Carotinoidkombinationen enthalten, Milchprodukten wie Joghurt, Milchmixgetränken oder Milchspeiseeis sowie Puddingpulvern, Backmischungen und Süßwaren, beispielsweise Fruchtgummis zuzugeben.

Gegenstand der Erfindung sind auch Nahrungsergänzungsmittel, Tierfuttermittel, Lebensmittel sowie pharmazeutische und kosmetische Zubereitungen, enthaltend die oben beschriebenen Carotinoid-Formulierungen von Gemischen aus β-Carotin, Lycopin und Lutein.

Unter Nahrungsergänzungspräparate sowie pharmazeutische Zubereitungen, die die erfindungsgemäße Carotinoidmischung enthalten, sind u.a. Tabletten, Dragees sowie Hart- und Weichgelatinekapseln zu verstehen. Bevorzugte Nahrungsergänzungspräparate sind Weichgelatinekapseln, in denen die Carotinoide als ölhaltige Suspension in den Kapseln vorliegen. Der Gehalt an Carotinoiden in diesen Kapseln liegt im Bereich von 0,5 bis 20 mg β-Carotin, 0,5 bis 20 mg Lycopin und 0,5 bis 20 mg Lutein, bevorzugt im Bereich von 1 bis 15 mg β-Carotin, 1 bis 15 mg Lycopin und 1 bis 10 mg Lutein, besonders bevorzugt im Bereich von 2 bis 10 mg β-Carotin, 2 bis 10 mg Lycopin und 1 bis 5 mg Lutein.

Kosmetische Zubereitungen, beispielsweise topisch anwendbare Zubereitungen wie Cremes, Lotionen enthalten die erfindungsgemäßen Carotinoid-Formulierungen bevorzugt in Form von Emulsionen; in oralen kosmetischen Zubereitungen wie z.B. Dragees können die Carotinoide ebenfalls als ölhaltige Suspension enthalten sein.

In den folgenden Beispielen wird die Herstellung der erfindungsgemäßen Formulierungen näher erläutert.

### Beispiel 1

### Herstellung einer ölhaltigen β-Carotin/Lycopin/Lutein Dispersion

In 1850 g eines mittelkettigen Triglycerids (Delios^{®} SK der Fa. Grünau) wurden 350 g kristallines β-Carotin, 180 g kristallines Lycopin und 70 g kristallines Lutein und 25 g α-Tocopheroleingerührt. Mit einem Blattrührer wurde solange nachgerührt, bis eine homogene Suspension vorlag. Die Mischung wurde in eine rührbare Vorlage umgefüllt, aus der die Suspension mittels Schlauchpumpe durch eine kontinuierlich betriebene Kugelmühle Dyno Mill KDL Spezial, deren Mahlbehälter mit 480 g Mahlkörpern Dragonit 25 (Durchmesser 850-1230 µm) gefüllt waren, gefördert wurde. Die Rührwellendrehzahl betrug 4500 U/min. Die aus der Mühle austretene feine Suspension wurde aufgefangen. Die mittlere Teilchegröße betrug ca. 15 µm.

### Beispiel 2

### Herstellung einer ölhaltigen β-Carotin/Lycopin/Lutein Dispersion

In einem Becherglas wurden bei Raumtemperatur 100 g einer 30 gew.-%-igen β-Carotin-Dispersion (Lucarotin^{®} 30 M der Fa. BASF), 50 g einer 20 gew.-%-igen Lutein-Dispersion (FloraGLO^{®} 20 der Fa. Kemin) und 100 g einer 10 gew.-%-igen Lycopin-Dispersion in Maiskeimöl (hergestellt in einer Dyno-Mill mit Glaskugeln 0,6-0,8 mm als Mahlkörper) intensiv mit einem Laborrührer vermischt.

### Beispiel 3

### Herstellung eines β-Carotin/Lycopin/Lutein Solubilisats

Eine auf 65°C vorgewärmte Suspension von 150 g kristallines β-Carotin, 50 g kristallines Lycopin und 25 g kristallines Lutein in 2500 g Polyoxyethylen (20) sorbitanmonostearat (Tween^{®} 60) wurde bei einem Durchsatz von 2,2 kg/h einer, in ein auf 190°C temperiertes Ölbad getauchten Heizschlange mit einem Innendurchmesser von 2 mm und einer Länge von 12 m zugeführt. Bei einer Temperatur von 164°C nach Austritt aus dem Wärmeaustauscher und nach einer Verweilzeit von 62 s wurde das Carotinoidgemisch im Emulgator gelöst. In einer anschließenden Mischkammer (nähere Einzelheiten zur Apparatur finden sich in der Beschreibung von EP-A-0 479 066) wurde die Carotinoid-Lösung mit 25°C warmen Wasser (Durchsatz: 5,4 kg/h) bei einer Mischungstemperatur von 62°C turbulent vermischt. Das Solubilisat wurde bei einem Druck von 20 bar über ein Druckbegrenzer-Ventil ausgetragen. Man erhielt eine dunkelrot gefärbte, mizellare Carotinoid-Lösung mit einem Carotinoid Gehalt (β-Carotin, Lycopin und Lutein im Verhältnis 1:0,33:0,17) von 2,0 Gew.-% und einer Mizellgröße von 20 nm.

### Beispiel 4

### Herstellung einer β-Carotin/Lycopin/Lutein Emulsion

a) In einem 100 ml Becherglas wurden 56 g destilliertes Wasser und 5,6 g 3-molare Natronlauge in einem Wasserbad auf 60°C erwärmt. Dann wurden 7 g Ascorbylpalmitat zugegeben und die Mischung mit einem Magnetrührer so lange gerührt, bis eine fast klare Lösung entstanden war.
b) 385 g Glycerin wurde in einem Wasserbad auf 60°C erwärmt und mit der nach a) hergestellten Lösung unter langsamen Rühren mit einem Magnetrührer gemischt.
c) 22 g kristallines β-Carotin, 8 g kristallines Lycopin, 4 g kristallines Lutein, 5,6 g α-Tocopherol und 166 g fraktioniertes Kokosöl (Miglyol^{®} 810, Fa. Hüls, Troisdorf) wurden in einem Ölbad, das auf 185°C temperiert wurde, über einen Zeitraum von 25 Minuten unter Rühren mit einem Paddelrührer erhitzt, wobei die Carotinoide in Lösung gingen.
d) Die gemäß c) hergestellte Lösung wurde mit einer Zahnkranzdispergiermaschine (Ultraturrax^{®}) über einen Zeitraum von 2 Minuten in die nach b) hergestellte Lösung einemulgiert. Die erhaltene Emulsion wurde auf 50°C abgekühlt und anschließend durch einmalige Passage über einen Hochdruckhomogenisator bei 800 bar homogenisiert.

Man erhielt eine Emulsion mit einer mittleren Teilchengröße von 0,2 µm und einem Carotinoidgehalt von 5,0 Gew.-%.

### Beispiel 5

### Herstellung eines β-Carotin/Lycopin/Lutein Trockenpulvers

600 g kristallines β-Carotin, 300 g kristallines Lycopin und 100 g kristallines Lutein wurden unter Stickstoffatmosphäre zu einer wäßrigen Lösung von 584 g Gelatine (Bloomzahl 240) und 100 g Natriumascorbat in 2800 g entgastem Wasser gegeben. Nach einstündigem Mahlen der Suspension mittels einer Kugelmühle, wurde die feingemahlene Suspension zu einer entgasten, wäßrigen Lösung von 1300 g Gelatine und 2044 g Saccharose gegeben. Die Lösung enthielt zusätzlich noch 9,5 g Ascorbylpalmitat als Emulgator und 14,3 g Tocopherol als Antioxidants. Nach intensiver Durchmischung wurde die erhaltene Dispersion nach an sich bekannter Weise sprühgetrocknet. Man erhielt ein Trockenpulver mit einem Carotinoidgehalt von 15 Gew.-%.

## Patentansprüche

1. Carotinoid-Formulierungen, enthaltend ein Gemisch aus ß-Carotin, Lycopin und Lutein, **dadurch gekennzeichnet, dass** der Phosphorgehalt der Formulierung kleiner 2,0 Gew.-%, bezogen auf die Gesamtmenge des Gemisches aus β-carotin, Lycopin und Lutein ist.

2. Carotinoid-Formulierungen nach Anspruch 1, enthaltend ein Gemisch aus 1 Teil β-Carotin, 0,05 bis 20 Teile Lycopin und 0,05 bis 20 Teile Lutein.

3. Carotinoid-Formulierungen nach Anspruch 1, enthaltend ein Gemisch aus 1 Teil β-Carotin, 0,3 bis 1,5 Teile Lycopin und 0,1 bis 0,5 Teile Lutein.

4. Carotinoid-Formulierungen nach einem der Ansprüche 1 bis 3, **dadurch gekennzeichnet, dass** die für die Carotinoid-Mischung verwendeten Einzelkomponenten jeweils eine Reinheit größer 75 Gew.-% aufweisen.

5. Carotinoid-Formulierungen nach einem der Ansprüche 1 bis 4, **dadurch gekennzeichnet, dass** es sich dabei um Lösungen, Solubilisate, Dispersionen oder Trockenpulver handelt.

6. Carotinoid-Formulierungen nach einem der Ansprüche 1 bis 5, **dadurch gekennzeichnet, dass** es sich dabei um ölhaltige Dispersionen handelt.

7. Carotinoid-Formulierungen nach einem der Ansprüche 1 bis 6, mit einem Carotinoid Gehalt von 0,1 bis 40 Gew.-%.

8. Carotinoid-Formulierungen nach einem der Ansprüche 1 bis 7, enthaltend zusätzlich 0,01 bis 40 Gew.-% eines oder mehrerer weiterer Wirkstoffe.

9. Verwendung der Carotinoid-Formulierungen, definiert gemäß einem der Ansprüche 1 bis 8, zur Herstellung von Nahrungsergänzungsmitteln sowie als Zusatz zu Tierfuttermitteln, Lebensmitteln, pharmazeutischen und kosmetischen Zubereitungen.

10. Verwendung von Carotinoid-Formulierungen nach Anspruch 9 zur Herstellung von Weichgelatinekapseln.

11. Nahrungsergänzungsmittel, Tierfuttermittel, Lebensmittel sowie pharmazeutische und kosmetische Zubereitungen, enthaltend Carotinoid-Formulierungen, definiert gemäß einem der Ansprüche 1 bis 8.

12. Weichgelatinekapseln, enthaltend Carotinoid-Formulierungen, definiert gemäß einem der Ansprüche 1 bis 8.

13. Weichgelatinekapseln nach Anspruch 12, enthaltend 0,5 bis 20 mg β-Carotin, 0,5 bis 20 mg Lycopin und 0,5 bis 20 mg Lutein.

## Claims

1. A carotenoid formulation comprising a mixture of β-carotene, lycopene and lutein, wherein the phosphorus content of the formulation is less than 2.0% of the total weight of the mixture of β-carotene, lycopene and lutein.

2. The carotenoid formulation according to claim 1, comprising a mixture of 1 part of β-carotene, 0.05 to 20 parts of lycopene and 0.05 to 20 parts of lutein.

3. The carotenoid formulation according to claim 1, comprising a mixture of 1 part of β-carotene, 0.3 to 1.5 parts of lycopene and 0.1 to 0.5 part of lutein.

4. The carotenoid formulation according to any of claims 1 to 3, wherein the individual components used for the carotenoid mixture each have a purity of greater than 75% by weight.

5. The carotenoid formulation according to any of claims 1 to 4, which is in the form of a solution, solubilizate, dispersion or dry powder.

6. The carotenoid formulation according to any of claims 1 to 5, which is in the form of an oil-containing dispersion.

7. The carotenoid formulation according to any of claims 1 to 6, having a carotenoid content of from 0.1 to 40% by weight.

8. The carotenoid formulation according to any of claims 1 to 7, additionally comprising 0.01 to 40% by weight of one or more other active substances.

9. The use of a carotenoid formulation as defined in any of claims 1 to 8, for producing food supplements and as additive to animal feeds, human foods, and pharmaceutical and cosmetic preparations.

10. The use of a carotenoid formulation according to claim 9 for producing soft gelatin capsules.

11. A food supplement, animal feed, human food or pharmaceutical or cosmetic preparation comprising a carotenoid formulation as defined in any of claims 1 to 8.

12. A soft gelatin capsule comprising a carotenoid formulation as defined in any of claims 1 to 8.

13. The soft gelatin capsule according to claim 12, comprising 0.5 to 20 mg of β-carotene, 0.5 to 20 mg of lycopene and 0.5 to 20 mg of lutein.

## Revendications

1. Formulations de caroténoïde, contenant un mélange de β-carotène, de lycopine et de lutéine, **caractérisées en ce que** la teneur en phosphore de la formulation est inférieure à 2,0 % en poids, par rapport à la quantité totale du mélange de β-carotène, de lycopine et de lutéine.

2. Formulations de caroténoïde suivant la revendication 1, contenant un mélange de 1 partie de β-carotène, de 0,05 à 20 parties de lycopine et de 0,05 à 20 parties de lutéine.

3. Formulations de caroténoïde suivant la revendication 1, contenant un mélange de 1 partie de β-carotène, de 0,3 à 1,5 partie de lycopine et de 0,1 à 0,5 partie de lutéine.

4. Formulations de caroténoïde suivant l'une des revendications 1 à 3, **caractérisées en ce que** les composants individuels utilisés pour le mélange de caroténoïde présentent chacun une pureté supérieure à 75 % en poids.

5. Formulations de caroténoïde suivant l'une des revendications 1 à 4, **caractérisées en ce qu'**il s'agit de solutions, de produits de solubilisation, de dispersions ou de poudres sèches.

6. Formulations de caroténoïde suivant l'une des revendications 1 à 5, **caractérisées en ce qu'**il s'agit de dispersions contenant de l'huile.

7. Formulations de caroténoïde suivant l'une des revendications 1 à 6, présentant une teneur en caroténoïde de 0,1 à 40 % en poids.

8. Formulations de caroténoïde suivant l'une des revendications 1 à 7, contenant en supplément 0,01 à 40 % en poids d'une ou de plusieurs autres substances actives.

9. Utilisation des formulations de caroténoïde, définies suivant l'une des revendications 1 à 8, pour la préparation de compléments alimentaires ainsi que comme additifs pour des fourrages pour animaux, des aliments, des compositions pharmaceutiques et cosmétiques.

10. Utilisation des formulations de caroténoïde suivant la revendication 9, pour la préparation de capsules de gélatine molle.

11. Compléments alimentaires, fourrages pour animaux, aliments, ainsi que compositions pharmaceutiques et cosmétiques, contenant des formulations de caroténoïde définies suivant l'une des revendications 1 à 8.

12. Capsules de gélatine molle, contenant des formulations de caroténoïde définies suivant l'une des revendications 1 à 8.

13. Capsules de gélatine molle suivant la revendication 12, contenant 0,5 à 20 mg de β-carotène, 0,5 à 20 mg de lycopine et 0,5 à 20 mg de lutéine.
